Europäisches Patentamt

European Patent Office

Office européen des brevets

(11) Publication number: **0 277 400 B1**

# EUROPEAN PATENT SPECIFICATION

(45) Date of publication of patent specification: **15.01.92**  (51) Int. Cl.⁵: **A61K 7/16, A61K 7/22**

(21) Application number: **87300920.3**

(22) Date of filing: **03.02.87**

(54) **Oral compositions.**

(43) Date of publication of application:
**10.08.88 Bulletin  88/32**

(45) Publication of the grant of the patent:
**15.01.92 Bulletin  92/03**

(84) Designated Contracting States:
**AT BE CH DE ES FR GB IT LI NL SE**

(56) References cited:
**FR-A- 2 440 189**
**GB-A- 2 122 491**

**"Handbuch der Kosmetika und Riechstoffe",
vol. 3, 1973, 2nd edition, Alfred Hüthig Ver-
lag, Heidelberg (DE); Band III: H.JANISTYN,
pp. 782-792**

**CHEMICAL ABSTRACTS, vol. 78, 1973, Co-
lumbus, OH (US); J.G.SARDI et al., p. 324, no.
82975u**

(73) Proprietor: **LION CORPORATION
3-7, Honjo 1-chome
Sumida-ku Tokyo(JP)**

(72) Inventor: **Suganuma, Nobuo
3-25 Yashiki 1-chome
Narashino-shi Chiba-ken(JP)**
Inventor: **Ishii, Shigeru
1-1-1113,Ohzima 6-chome
Kohtoh-ku Tokyo(JP)**
Inventor: **Gomi, Tetsuo
24-7, Kouyama 3-chome
Nerima-ku Tokyo(JP)**

(74) Representative: **Ellis, John Clifford Holgate et
al
MEWBURN ELLIS & CO. 2/3 Cursitor Street
London EC4A 1BO(GB)**

Rank Xerox (UK) Business Services

## Description

This invention relates to an oral composition having tranexamic acid and/or a salt thereof blended therein, and more particularly, to such an oral composition whose bitter and pungent tastes due to the inclusion of tranexamic acid and its salt are mitigated to provide a pleasant feel in use and which experiences controlled coloring during an extended period of storage and thus exhibits excellent shelf stability.

It is well known in the art to blend tranexamic acid or its salts in oral compositions, typically dentifrices,as an active ingredient effective for preventing paradental diseases. Oral compositions having tranexamic acid or its salts blended therein, however, taste bitter and hot or pungent and will be undesirably colored during an extended period of shelf storage.

In UK Patent No. 207309 by the same applicant as the present invention, it was proposed to mitigate the bitter taste of tranexamic acid-containing oral compositions by blending carvone therein. It was also disclosed that the shelf stability of tranexamic acid-containing oral compositions is improved by blending carvone in combination with l-menthol.

It is believed that discoloration of oral compositions having tranexamic acid or its salts blended therein is caused by reaction of tranexamic acid or its salts with an aldehyde group in a flavor (see Japanese Patent Publication No. 55-5484 and Japanese Patent Application Laid-Open No. 56-122308). The same applicant as the present invention proposed to blend a specific aldehyde flavor in an oral composition having tranexamic acid or its salts blended therein (see UK Patent No. 2122491), based on the discovery that the specific aldehyde flavor is effective in preventing discoloration of the oral composition having tranexamic acid or its salts blended therein.

However, it is sometimes desired to blend in oral compositions aldehyde flavors which would probably cause discoloration of tranexamic acid-containing oral composition, for example, citral, atrans-2-hexenal, and trans-2-octenal because the aldehyde flavors impart a more pleasant feel to the oral compositions. It is thus needed to prevent discoloration of oral compositions having such aldehyde flavors blended therein along with tranexamic acid and its salts and to control the bitter and pungent tastes characteristic of tranexamic acid and its salts.

A primary object of the present invention is to provide a novel and improved oral composition having tranexamic acid and/or a salt thereof blended therein, which tastes less bitter or pungent, thus giving a pleasant feel in use, and experiences controlled coloring during an extended period of storage, thus possessing excellent shelf stability.

Continuing our investigations to improve the feel and shelf stability of oral compositions having tranexamic acid or its salts blended therein and to prevent discoloration of such tranexamic acid-containing oral compositions having incorporated therein aldehyde flavors probably causing discoloration of tranexamic acid or its salts, for example, citral, tans-2-hexenal, and trans-2-octenal, we have discovered that menthone, isomethone and pulegone have the unique effect of improving the inherent bitter and pungent tastes of oral compositions having tranexamic acid or its salts blended therein, and prohibiting discoloration of the tranexamic acid-containing oral compositions having incorporated therein an aldehyde flavor which will otherwise cause discoloration of such compositions.

As previously described, carvone can improve the bitter taste due to tranexamic acid or its salts, but cannot prevent discoloration of tranexamic acid or its salts when used alone. Combination of carvone with l-menthol provides a synergistic effect of preventing discoloration. Without any assisting agent, menthone, isomenthone and pulegone can mitigate the bitter and pungent tastes of tranexamic acid or its salts to impart a favorable feel in use, and have such a specific interaction with an aldehyde flavor including citral, trans-2-hexenal and trans-2-octenal as to cause no discoloration of tranexamic acid-containing oral compositions even when they contain any of these aldehyde flavors which will otherwise cause discoloration of such composition. By formulating menthone, isomenthone or pulegone having such improved discoloration control, it is possible to blend any aldehyde flavors which may cause discoloration in tranexamic acid-containing oral compositions without any problem or rather with an improved feel in use.

Therefore, the present invention provides an oral composition comprising
tranexamic acid and/or a salt thereof,
an aldehyde flavor, and
at least one cyclic ketone selected from the group consisting of menthone, isomenthone, and pulegone.

The amount of tranexamic acid and/or a salt thereof blended in the oral composition preferably ranges from 0.01 to 5%, more preferably from 0.01 to 1% by weight of the total weight of the composition.

The aldehyde flavor used in the invention is selected from aldehyde flavors which would probably cause discoloration of tranexamic acid-containing oral compositions. Examples of such aldehyde flavors include citral, trans-2-hexenal, trans-2-octenal, and trans-2-pentenal. The flavors may be used alone or in combination of two or more.

A compatible aldehyde flavor which would not cause discoloration of tranexamic acid-containing oral compositions as disclosed in UK Patent No. 207309 may also be blended.

The amount of the aldehyde flavor blended in the oral composition preferably ranges from 0.001 to 10%, more preferably from 0.005 to 5% by weight of the total weight of the composition for the purpose of imparting a pleasant feel.

In addition to the above-mentioned flavors, the oral compositions of the present invention may further contain other flavors such as carvone, l-menthol, anethole, methyl salicylate, etc. alone or in admixture of two or more.

According to the present invention, a specific cyclic ketone selected from menthone, isomenthone and pulegone is blended in the oral composition in order to mitigate the bitter and pungent tastes inherent to tranexamic acid or its salts and to prevent discoloration of the composition which is otherwise caused by the aldehyde flavor.

The amount of the cyclic ketone blended in the oral composition preferably ranges from 0.0001 to 1%, more preferably from 0.001 to 0.1% by weight of the total weight of the composition.

The oral compositions according to the present invention encompass a variety of oral compositions, for example, dentifrices such as toothpaste, tooth powder and liquid dentifrice, liquid oral refreshers such as mouthwash, and solid oral refreshers such as troches, chewing gums and oral pastes. In addition to the above-mentioned ingredients, any suitable ingredients may be selected and blended in the oral compositions of the present invention depending on their type.

More specifically, for dentifrices, abrasives may be blended in an amount of 20 to 90% by weight (for toothpastes, 20 to 60% by weight), including dicalcium phosphate dihydrate and anhydride, calcium primary phosphate, calcium tertiary phosphate, calcium carbonate, calcium pyrophosphate, aluminum hydroxide, alumina, silicic anhydride, silica, silica gel, aluminosilicate, aluminum silicate, insoluble sodium metaphosphate, magnesium tertiary phosphate, magnesium carbonate, calcium sulfate, polymethyl methacrylate, bentonite, zirconium silicate and mixtures thereof.

For pasty compositions, for example, toothpastes, a binder may be blended in an amount of 0.3 to 5% by weight, including synthetic binders such as carrageenan, cellulose derivatives such as sodium carboxymethylcellulose, methyl cellulose, hydroxyethylcellulose, sodium carboxymethylhydroxyethylcellulose, alkali metal alginates such as sodium alginate, propylene glycol alginate, gums such as xanthane gum, tragacanth gum, karaya gum and gum arabic, polyvinyl alcohol, sodium polyacrylate, carboxyvinyl polymer, polyvinyl pyrrolidone, and inorganic binders such as silica gel, aluminum silicate gel, veegum, laponite and mixtures thereof.

In preparing pasty and liquid oral compositions such as toothpastes and mouthwashes, a humectant may be blended in an amount of 10 to 70% by weight, including sorbitol, glycerin, ethylene glycol, propylene glycol, 1,3-butylene glycol, polyethylene glycol, polypropylene glycol, xylitol, maltitol and lactitol and mixtures thereof.

There may also be included anionic surfactants such as water-soluble salts of higher alkyl sulfates (e.g., sodium lauryl sulfate), sodium salts of higher fatty acids, water-soluble salts of sulfonated monoglycerides of higher fatty acids having 10 to 18 carbon atoms in the fatty acid group (e.g., sodium lauryl monoglyceride sulfonate and sodium coconut monoglyceride sulfonate), higher fatty acid sodium monoglyceride monosulfates, olefin sulfonates, paraffin sulfonates; nonionic surfactants such as fatty acid mono- and diethanol amides (e.g., lauroyl mono- and diethanol amides), stearyl monoglyceride, sucrose fatty acid esters having 12 to 18 carbon atoms in the fatty acid group (e.g., sucrose mono laurate and dilaurate), lactose fatty acid esters, lactitol fatty acid esters, maltitol fatty acid esters, stearic acid monoglyceride, polyoxyethylene sorbitan monolaurate, polyoxyethylene-hardened castor oil, condensates of sorbitan monostearate with approximately 60 moles of ethylene glycol, condensates of ethylene oxide with propylene oxide and their derivatives (e.g., polyoxyethylene polyoxypropylene monolauryl ester); and amphoteric surfactants such as those of betaine type alone or in admixture of two or more in an amount of 0 to 10% by weight, preferably 0.1 to 5% by weight.

Also includable in the oral compositions according to the present invention are sweeteners such as sodium saccharin, stevioside, neohesperidin dihydrocalcone, glycyrrhizin, perillartin, thaumatin, fructose and sodium cyclamate in an amount of 0 to 1% by weight, preferably 0.01 to 0.5% by weight; an antiseptic agent such as p-hydroxymethylbenzoic acid, p-hydroxyethylbenzoic acid, p-hydroxypropyl benzoic acid, p-hydroxybutylbenzoic acid, sodium benzoate and lower fatty acid monoglycerides; titanium dioxide, ethanol, liquid paraffin, coloring matter, and other additives. For example, toothpastes may be prepared by kneading the desired ingredients selected from the foregoing ingredients with a proper amount of water.

Other types of oral compositions may also be prepared in a conventional manner by selecting commonly used ingredients for the respective types.

In this case, the pH of pasty and liquid oral compositions generally falls in the range of from 5 to 10 although not particularly limited thereto.

In the practice of the invention, one or more active ingredients may also be blended. Antiphonal agentshave already been mentioned. Others may include, for example, alkali metal monofluorophosphates such as sodium monofluorophosphate and potassium monofluorophosphate, fluorides such as sodium fluoride and stannous fluoride, chlorohexidine salts, aluminum chlorohydroxylallantoin, dihydrocholesterol, glycyrrhizin salts, glycyrrhetinic acid, glycerophosphate, chlorophyll, sodium chloride, caropeptide, quaternary ammonium compounds and water-soluble inorganic phosphoric acid compounds.

Because of having menthone, isomenthone or pulegone blended therein, the oral compositions of the present invention exhibit minimized bitter and pungent tastes due to tranexamic acid or its salts and minimized discoloration due to an aldehyde flavor which will otherwise cause discoloration of the oral composition, thus having a more pleasant feel in use and an extended shelf stability.

In the present disclosure, all parts and percents are by weight unless otherwise stated.

Several experiments are given below to demonstrate the effectiveness of the present invention.

Experiment 1

Mouthwashes having the following composition were prepared.

| Mouthwash composition | % by weight |
|---|---|
| Tranexamic acid | 0.1 |
| 85% Glycerin | 10 |
| Ethanol | 10 |
| Polyoxyethylene (60) hardened castor oil | 1 |
| Saccharin sodium | 0.05 |
| l-Menthol | 0.2 |
| Cyclic ketone shown in Table 1 | 0.2 |
| Water | balance |
| | 100.0% |

The mouthwashes were subjected to a sensory tests as to bitter and hot tastes. Using a panel of ten specialists, evaluation was made by examining the taste of samples into the following five grades, and classifying the average according to the following criterion. The results are shown in Table 1.

Grade

The following five grades were given in comparison with a control mouthwash, that is, mouthwash having the same composition as above except that the flavor shown in Table 1 was omitted.
5: markedly improved
4: improved
3: somewhat improved
2: not improved
1: rather worse

Criterion

E: averaging between 5 and 4
G: averaging between 4 and 3
F: averaging between 3 and 2
B: averaging between 2 and 1

Table 1

| Flavor | Bitter | Hot |
|---|---|---|
| Menthone | E | G |
| Isomenthone | E | G |
| Piperitone | E | G |
| Pulegone | E | G |
| Piperitenone | G | F |
| Cis-jasmone | E | G |
| 3-Methylcyclohexanone | G | F |
| Cryptone | G | G |
| Acetophenone | G | F |
| Camphor | G | G |
| Anisylacetone | F | F |
| $\beta$-Methylionone | B | F |

As seen from the data of Table 1, particularly menthone, isomenthone, piperitone, pulegone, and cis-jasmone make a noticeable improvement in the bitter and hot tastes of tranexamic acid-containing oral compositions.

Experiment 2

Mouthwashes having the following composition, that is, having blended the cyclic ketone shown in Table 2, were prepared.

| Mouthwash composition | % by weight |
|---|---|
| Tranexamic acid | 0.2 |
| 85% Glycerin | 12.0 |
| Ethanol | 12.0 |
| Polyoxyethylene (60) hardened castor oil | 4.5 |
| Saccharin sodium | 0.5 |
| Citral | 0.4 |
| Cyclic ketone shown in Table 2 | 0.4 |
| Water | balance |
| | 100.0% |

The mouthwashes were charged in transparent glass bottles and shelf stored for 5 days at 50°C whereupon they were evaluated for discoloration. Evaluation was made in comparison with a control mouthwash, that is, mouthwash having the same composition as above except that the cyclic ketone shown in Table 2 was omitted. The results are shown in Table 2. Samples with "NO" are those samples exhibiting controlled discoloration.

5

Table 2

| Flavor | Discoloration |
|--------|---------------|
| Menthone | NO |
| Isomenthone | NO |
| Piperitone | Discolored |
| Pulegone | NO |
| Piperitenone | Discolored |
| Cis-jasmone | Discolored |
| 3-Methylcyclohexanone | Discolored |
| Cryptone | Discolored |
| Acetophenone | No |
| Camphor | NO |
| Anisylacetone | NO |

As seen from the data of Table 2, menthone, isomenthone, pulegone, acetophenone, camphor, and anisyl acetone are effective in controlling discoloration due to blending of citral.

From the combined results of Experiments 1 and 2, menthone, isomenthone and pulegone are recognized as outstandingly mitigating the bitter and hot tastes of tranexamic acid and controlling discoloration of tranexamic acid-containing oral compositions which is otherwise caused by blending of citral.

Experiment 3

There were prepared mouthwashes having the same composition as shown in Experiment 2 except that 0.4% of menthone as the cyclic ketone and aldehyde flavors shown in Table 3 were blended. The mouthwashes were examined for discoloration by the same procedure as in Experiment 2. The results are shown in Table 3.

Table 3

| Aldehyde flavor | Discoloration |
|-----------------|---------------|
| Citral | NO |
| Trans-2-hexenal | NO |
| Trans-2-octenal | NO |

As seen from the data of Table 3, menthone makes such a specific interaction with citral, trans-2-hexenal and trans-2-octenal as to control discoloration of tranexamic acid-containing oral compositions which is otherwise caused by blending of these aldehyde flavors.

Similar results were obtained with isomenthone and pulegone used instead of menthone.

Examples of the present invention are given below by way of illustration and not by way of limitation.

6

| Example 1: Toothpaste | |
|---|---|
| Ingredient | % by weight |
| Silicic anhydride | 25.0 |
| Sodium carboxymethyl cellulose | 0.8 |
| Sodium alginate | 0.4 |
| Sorbitol | 30.0 |
| Glycerin | 10.0 |
| Propylene glycol | 3.0 |
| Sodium lauryl sulfate ($C_{10}$-$C_{16}$)* | 1.5 |
| Sodium lauroyl sarcosinate | 0.3 |
| Polyoxyethylene sorbitan monolaurate | 0.1 |
| Tranexamic acid | 0.1 |
| -aminocaproic acid | 0.05 |
| Allantoinate | 0.1 |
| Sodium benzoate | 0.1 |
| Saccharin sodium | 0.1 |
| Flavor blend No. 1 | 0.8 |
| Distilled water | balance |
| | 100.0% |

* Sodium lauryl sulfate ($C_{10}$-$C_{16}$) is a mixture of sodium alkyl sulfates wherein at least 98% of the overall carbon atom number distribution consists of ($C_{10}$-$C_{16}$) alkyls in the following proportion:

$C_{10}$: 0-20%

$C_{12}$: 50-80%

$C_{14}$: 10-30%

$C_{16}$: 0-15%

| Formulation of flavor blend No. 1 | |
|---|---|
| Ingredient | Parts by weight |
| l-Menthol | 30.0 |
| Peppermint oil | 30.0 |
| Spearmint oil | 1.0 |
| Anethole | 6.0 |
| Eugenol | 0.1 |
| Trans-2-hexenal | 1.0 |
| Cardamom oil | 1.0 |
| Strawberry flavor | 2.5 |
| Menthone | 1.0 |
| Isomenthone | 5.0 |
| Cis-jasmone | 1.0 |
| Ethanol | 1.4 |
| | 80.0 |

| Example 2: Toothpaste | |
|---|---|
| Ingredient | % by weight |
| Calcium hydrogenphosphate dihydrate | 25.0 |
| Aluminum hydroxide | 25.0 |
| Silicic anhydride | 2.0 |
| Sodium carboxymethyl cellulose | 0.9 |
| Carrageenan | 0.3 |
| Sorbitol | 20.0 |
| Polyethylene glycol | 3.0 |
| Sodium lauryl sulfate | 1.0 |
| Myristoyl diethanolamide | 0.5 |
| Tranexamic acid | 0.3 |
| Chlorohexidine hydrochloride | 0.01 |
| Sodium monofluorophosphate | 0.76 |
| Dipotassium glycyrrhetinate | 0.1 |
| Paraben | 0.1 |
| Saccharin sodium | 0.1 |
| Flavor blend No. 2 | 0.8 |
| Distilled water | balance |
| | 100.0% |

| Formulation of flavor blend No. 2 | |
|---|---|
| Ingredient | Parts by weight |
| l-Menthol | 30.0 |
| Peppermint oil | 8.0 |
| Anethole | 3.0 |
| Cineole | 2.0 |
| Methyl salicylate | 18.0 |
| Benzaldehyde | 5.0 |
| Cardamom oil | 1.0 |
| Strawberry flavor | 1.0 |
| Citral | 1.0 |
| Menthone | 5.0 |
| Isomenthone | 2.0 |
| Piperitone | 1.0 |
| Pulegone | 1.0 |
| 3-Methylcyclohexanone | 0.1 |
| Cryptone | 1.0 |
| Ethanol | 0.9 |
| | 100.0 |

8

| Example 3: Toothpaste | |
|---|---|
| Ingredient | % by weight |
| Aluminum hydroxide | 45.0 |
| Silicic anhydride | 3.0 |
| Sodium carboxymethyl cellulose | 1.4 |
| Sorbitol | 10.0 |
| Glycerin | 10.0 |
| Polyethylene glycol | 3.0 |
| Sodium lauryl sulfate ($C_{10}$-$C_{16}$)[*] | 1.2 |
| Sucrose monolaurate | 0.3 |
| Tranexamic acid | 0.1 |
| Chlorohexidine gluconate | 0.01 |
| Dipotassium glycirrhetinate | 0.1 |
| Saccharin sodium | 0.1 |
| Flavor blend No. 3 | 0.81 |
| Distilled water | balance |
| | $\overline{100.0\%}$ |

[*] see Example 1

| Formulation of flavor blend No. 3 | |
|---|---|
| Ingredient | Parts by weight |
| l-Menthol | 30.0 |
| Peppermint oil | 10.0 |
| Anethole | 5.0 |
| Cineole | 20.0 |
| Citral | 4.0 |
| Cardamom oil | 3.0 |
| Laurel oil | 1.0 |
| Pulegone | 5.0 |
| Cis-jasmone | 1.0 |
| Camphor | 1.0 |
| Ethanol | 1.0 |
| | $\overline{81.0}$ |

| Example 4: Toothpaste | |
|---|---|
| Ingredient | % by weight |
| Calcium hydrogenphosphate dihydrate | 25.0 |
| Calcium hydrogenphosphate anhydride | 25.0 |
| Silicic anhydride | 2.0 |
| Sodium carboxymethyl cellulose | 1.0 |
| Carrageenan | 0.3 |
| Sorbitol | 10.0 |
| Glycerin | 15.0 |
| Propylene glycol | 3.0 |
| Sodium lauryl sulfate ($C_{10}$-$C_{16}$)* | 1.5 |
| Polyoxyethylene sorbitan monolaurate | 0.1 |
| Tranexamic acid | 0.3 |
| Stearyl glycirrhetinate | 0.1 |
| Saccharin sodium | 0.15 |
| Flavor blend No. 4 | 0.6 |
| Distilled water | balance |
| | $\overline{100.0\%}$ |

* see Example 1

| Formulation of flavor blend No. 4 | |
|---|---|
| Ingredient | Parts by weight |
| l-Menthol | 8.0 |
| Hexyl aldehyde | 0.05 |
| Trans-2-hexenal | 0.05 |
| Cardamom oil | 0.05 |
| Melon flavor | 50.0 |
| Laurel oil | 0.05 |
| Menthone | 0.5 |
| Cis-jasmone | 0.5 |
| Ethanol | 0.8 |
| | $\overline{60.0}$ |

| Example 5: Mouthwash | |
|---|---|
| Ingredient | % by weight |
| Glycerin | 10.0 |
| Ethanol | 10.0 |
| Saccharin sodium | 0.3 |
| Polyoxyethylene-hardened castor oil | 1.0 |
| Tranexamic acid | 0.1 |
| Chlorohexidine hydrochloride | 0.01 |
| Flavor blend No. 5 | 0.68 |
| Distilled water | balance |
| | $\overline{100.0\%}$ |

EP 0 277 400 B1

| Formulation of flavor blend No. 5 | |
| --- | --- |
| Ingredient | Parts by weight |
| l-Menthol | 20.0 |
| Peppermint oil | 0.3 |
| Anethole | 2.0 |
| Cineole | 1.4 |
| Eugenol | 1.0 |
| Methyl salicylate | 40.0 |
| Citral | 1.0 |
| Strawberry flavor | 0.1 |
| Menthone | 0.1 |
| Cis-jasmone | 0.1 |
| Ethanol | 2.0 |
| | 68.0 |

## Claims

1. An oral composition comprising tranexamic acid and/or a salt thereof and an aldehyde flavor which would normally cause discoloration of a tranexamic acid-containing oral composition, characterized in that the composition further contains at least one cyclic ketone selected from menthone, isomenthone and pulegone.

2. An oral composition according to claim 1 wherein the amount of tranexamic acid and/or a salt thereof blended ranges from 0.01 to 5% by weight of the total weight of the composition.

3. An oral composition according to claim 1 or 2 wherein the amount of the aldehyde flavor blended ranges from 0.001 to 10% by weight of the total weight of the composition.

4. An oral composition according to any one of claims 1 to 3 wherein the amount of the cyclic ketone blended ranges from 0.0001 to 1% by weight of the total weight of the composition.

5. An oral composition according to any preceding claim wherein the aldehyde flavor is selected from citral, trans-2-hexenal, trans-2-octenal and trans-2-pentenal.

## Revendications

1. Composition orale comprenant de l'acide tranexamique et/ou son sel et un arome d'aldéhyde qui provoquerait normalement une décoloration d'une composition orale comprenant de l'acide tranexamique, caractérisée en ce que la composition contient de plus au moins une cétone cyclique choisie parmi la menthone, l'isomenthone et la pulégone.

2. Composition orale selon la revendication 1 où la quantité de l'acide tranexamique et/ou son sel mélangé est comprise entre 0,01 et 5% en poids du poids total de la composition.

3. Composition orale selon la revendication 1 ou 2 où la quantité de l'arôme d'aldéhyde en mélange est comprise entre 0,001 et 10% en poids du poids total de la composition.

4. Composition orale selon l'une quelconque des revendications 1 à 3, où la quantité de l'acétone cyclique en mélange est comprise entre 0,0001 et 1% en poids du poids total de la composition.

5. Composition orale selon toute revendication précédente où l'arôme d'aldéhyde est choisi parmi le citral, le trans-2-hexénal, le trans-2-octénal et le trans-2-penténal.

## Patentansprüche

11

1. Orale Zusammensetzung, umfassend Tranexamsäure und/oder ein Salz davon und einen Aldehydgeschmacksstoff, der üblicherweise Verfärbung einer Tranexamsäure-hältigen oralen Zusammensetzung verursachen würde, dadurch gekennzeichnet, daß die Zusammensetzung weiters zumindest ein aus Menthon, Isomenthon und Pulegon ausgewähltes zyklisches Keton enthält.

2. Orale Zusammensetzung nach Anspruch 1, worin die Menge an beigemengter Tranexamsäure und/oder einem Salz davon im Bereich von 0,01 bis 5 Gew-% des Gesamtgewichts der Zusammensetzung liegt.

3. Orale Zusammensetzung nach Anspruch 1 oder 2, worin die Menge an beigemengtem Aldehydgeschmacksstoff im Bereich von 0,001 bis 10 Gew-% des Gesamtgewichts der Zusammensetzung liegt.

4. Orale Zusammensetzung nach einem der Ansprüche 1 bis 3, worin die Menge an beigemengtem zyklischen Keton im Bereich von 0,0001 bis 1 Gew% des Gesamtgewichts der Zusammensetzung liegt.

5. Orale Zusammensetzung nach einem der vorhergehenden Ansprüche, worin der Aldehydgeschmacksstoff aus Citral, Trans-2-hexenal, Trans-2-octenal und Trans-2-pentenal ausgewählt ist.